Europäisches Patentamt

European Patent Office

Office européen des brevets

(19)

(11) Veröffentlichungsnummer: **0 123 836**
**B1**

(12) # EUROPÄISCHE PATENTSCHRIFT

(45) Veröffentlichungstag der Patentschrift:
**27.01.88**

(51) Int. Cl.⁴: **A 61 M 5/14**

(21) Anmeldenummer: **84102563.8**

(22) Anmeldetag: **09.03.84**

(54) **Verfahren zur Auslösung von Voralarm bei einer Infusionsspritzenpumpe.**

(30) Priorität: **22.04.83 DE 3314664**

(43) Veröffentlichungstag der Anmeldung:
**07.11.84 Patentblatt 84/45**

(45) Bekanntmachung des Hinweises auf die Patenterteilung:
**27.01.88 Patentblatt 88/4**

(84) Benannte Vertragsstaaten:
**FR GB IT SE**

(56) Entgegenhaltungen:
**US - A - 3 858 581**
**US - A - 4 176 349**

(73) Patentinhaber: **Intermedicat GmbH, Gerliswilstrasse 45, CH-6020 Emmenbrücke (CH)**

(72) Erfinder: **Rosskopf, Gerhard, Dr., Heiligenbergstrasse 29, D-3501 Fuldabrück-Dörnhagen (DE)**

(74) Vertreter: **von Kreisler, Alek et al, Patentanwälte Von Kreisler-Schönwald-Fues-Keller Selting-Werner Deichmannhaus am Hauptbahnhof, D-5000 Köln 1 (DE)**

## Beschreibung

Die Erfindung betrifft ein Verfahren zur Auslösung von Voralarm bei einer Infusionsspritzenpumpe nach dem Oberbegriff des Patentanspruchs.

Es ist bekannt, zum Injizieren von Medikamenten in den Körper von Patienten Spritzenpumpen zu verwenden, die die das Medikament enthaltende Infusionsspritze kontinuierlich ausdrücken. Derartige Spritzenpumpen weisen einen Halter auf, an dem der Spritzenzylinder befestigt wird, und einen relativ zu dem Halter bewegbaren Schlitten, an dem der Spritzenkolben befestigt wird. Durch einen Motorantrieb wird der Schlitten kontinuierlich bewegt, wodurch infolge des Vorschiebens des Spritzenkolbens das Injektat aus der Spritze herausgedrückt wird.

Bevor die Infusionsspritze geleert ist, muss ein Alarmsignal erzeugt werden, das dem Arzt bzw. der Krankenschwester angibt, dass die Spritze in Kürze leer sein wird, so dass vorbereitende Massnahmen getroffen werden können, um beispielsweise die entleerte Spritze gegen eine gefüllte neue Spritze auszuwechseln. Hierzu ist bei einem bekannten Verfahren, von dem der Oberbegriff des Patentanspruchs ausgeht, ein Sensor vorgesehen, der bei einer bestimmten Position des Schlittens anspricht, bevor der Spritzenkolben seine Endstellung erreicht hat.

Infusionspumpen werden nicht nur in Verbindung mit einer bestimmten Spritzengrösse bzw. einem festen Spritzentyp verwandt, sondern sie müssen für unterschiedliche Spritzen einsetzbar sein. Auch die Vorschubgeschwindigkeit des Schlittens sollte einstellbar sein, um unterschiedliche Infusionsgeschwindigkeiten erzielen zu können. Die Infusionsgeschwindigkeiten liegen beispielsweise im Bereich von 0,1 bis 99,9 ml/h. Dieser weite Bereich von Infusionsgeschwindigkeiten hat zur Folge, dass die Zeit, die zwischen der Aktivierung des Sensors und der vollständigen Entleerung der Spritze vergeht, sehr unterschiedlich sein kann. Normalerweise wird der Sensor so angebracht, dass er betätigt wird, wenn bei der maximalen Infusionsgeschwindigkeit noch drei Minuten vergehen, bis die Infusionsspritze entleert ist. Ist die eingestellte Infusionsgeschwindigkeit niedriger als die maximale Infusionsgeschwindigkeit, dann wird der Voralarm sehr frühzeitig ausgelöst, so dass er das Bevorstehen der Entleerung nicht mehr wirksam angibt. Ein Voralarm, der nicht in einer festen Zeitbeziehung zu dem Ereignis steht, das er ankündigen soll, ist aber praktisch nutzlos und sogar störend.

Der Erfindung liegt die Aufgabe zugrunde, ein Verfahren der eingangs genannten Art anzugeben, das die Auslösung des Voralarms unabhängig von der jeweils eingestellten Infusionsrate um eine festgelegte und definierte Zeitspanne vor dem Infusionsende ermöglicht.

Die Lösung dieser Aufgabe erfolgt erfindungsgemäss mit den im kennzeichnenden Teil des Patentanpruchs angegebenen Merkmalen.

Nach der Erfindung wird im Anschluss an die Aktivierung des Sensors die Restinfusionszeit ermittelt, die bis zum vollständigen Entleeren der Infusionsspritze noch erforderlich ist. Danach findet gewissermassen eine Zeitzählung statt und nach Ablauf der berechneten Zeit wird um die vorgegebene Zeitspanne vor Infusionsende das Alarmsignal erzeugt. Die Erzeugung des Alarmsignals geschieht somit nicht wegabhängig wie bei den bekannten Verfahren und auch nicht ausschliesslich zeitabhängig, sondern bis zur Aktivierung des Sensors wegabhängig und im Anschluss an die Aktivierung des Sensors zeitabhängig, wobei die Infusionsrate bzw. die Bewegungsgeschwindigkeit des Schlittens, berücksichtigt wird. Wenn die Zeitspanne, die das Alarmsignal gegenüber dem Infusionsende vorverlegt ist, z.B. drei Minuten beträgt, so wird das Alarmsignal bei jeder eingestellten Infusionsgeschwindigkeit stets drei Minuten vor Infusionsende erzeugt. Diese Zeitspanne reicht für das Klinikpersonal aus, um die erforderlichen Vorbereitungen für das Infusionsende zu treffen. Sie ist andererseits nicht so lang, dass die betreffende Person die Infusionsspritzenpumpe wieder verlassen würde, um zwischenzeitlich noch anderweitige Tätigkeiten durchzuführen.

Die Zeitzählung, die erforderlich ist, um, beginnend mit der Aktivierung des Sensors, den Zeitpunkt der Alarmauslösung zu bestimmen, kann durch Zählen eines Zeittaktes entweder in einer diskreten Zählschaltung oder im Zuge des Mikroprozessor-Programms erfolgen. Die Berechnung der Restinfusionszeit erfolgt durch den Mikroprozessor.

Im folgenden wird unter Bezugnahme auf die Zeichnungen ein Ausführungsbeispiel der Erfindung näher erläutert.

Es zeigen:

Fig. 1 eine schematische Darstellung einer Infusionsspritzenpumpe und
Fig. 2 ein Flussdiagramm des Rechnerprogramms zur Bestimmung der Zeitpunkte des Voralarms und der Endabschaltung.

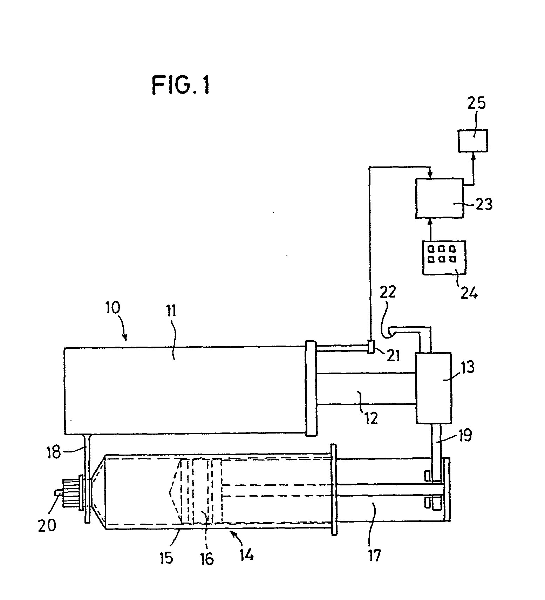

Die in Fig. 1 dargestellte Infusionsspritzenpumpe 10 weist ein Gehäuse 11 auf, das einen (nicht dargestellten) Antriebsmotor enthält. Der Antriebsmotor treibt eine Spindel 12, die aus einer Stirnseite des Gehäuses 11 herausragt und an deren äusserem Ende der Schlitten 13 befestigt ist.

Die Infusionsspritze 14 besteht aus dem Spritzenzylinder 15 und dem Spritzenkolben 16 mit der Kolbenstange 17. Das vordere Ende des Spritzenzylinders 15 ist in einen Halter 18 eingesetzt, der an dem Gehäuse 11 der Infusionsspritzenpumpe 10 befestigt ist. Das rückwärtige Ende der Kolbenstange 17 ist in einen weiteren Halter 19 eingesetzt, der am Schlitten 13 angebracht ist. Wird der Schlitten 13 in Richtung auf das Gehäuse 10 bewegt, wird der Spritzenkolben 16 im Spritzenzylinder 15 vorgeschoben und das Injektat wird durch den Spritzenauslass 20 hindurch ausgeschoben. Der Spritzenauslass ist über einen (nicht dargestellten) Schlauch mit dem Patienten verbunden.

In Fig. 1 ist schematisch ein Sensor 21 dargestellt, der an dem Gehäuse 11 befestigt ist und bei einer bestimmten Vorschubstellung des Schlittens 13 von einem an dem Schlitten befestigten Aktivator 22 aktiviert wird. Der Sensor 21 kann beispielsweise ein Mikroschalter sein, der von einem Kontaktnocken betätigt wird, eine Lichtschranke, ein Magnetschalter o.dgl.

Der Antrieb der Infusionsspritzenpumpe wird von dem Mikroprozessor 23 gesteuert. Über eine Eingabetastatur 24 werden dem Mikroprozessor die erforderlichen Daten, wie Spritzenvolumen, Infusionsgeschwindigkeit usw., eingegeben. Der Mikroprozessor 23 empfängt ausserdem das Signal des Sensors 21. Ferner ist an dem Mikroprozessor 23 der Alarmgeber 25 angeschlossen, der in noch zu erläuternder Weise betätigt wird. Der Alarmgeber kann ein optischer und/oder akustischer Alarmgeber sein.

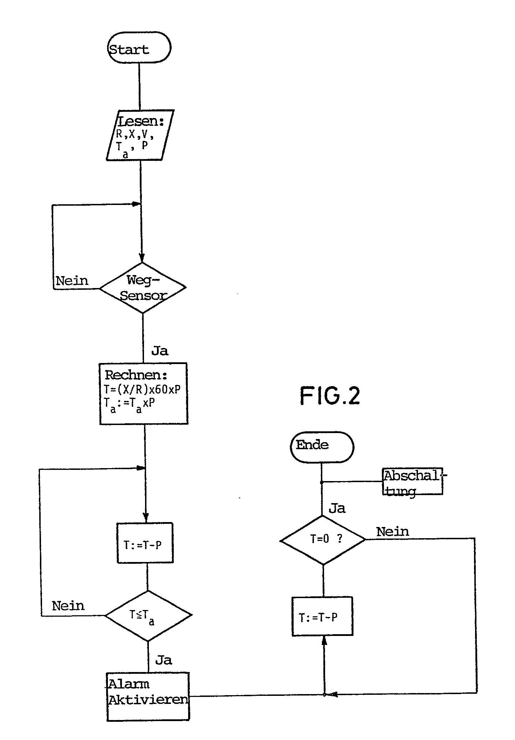

Gemäss Fig. 2 erfolgt nach dem Startbefehl, der zu Beginn des Infusionsvorganges dem Mikroprozessor mitgeteilt wird, das Einlesen der Infusionsgeschwindigkeit R (ml/min), des Restvolumens X (ml), das sich bei Aktivierung des Sensors noch in der Infusionsspritze befindet, des Gesamtinfusionsvolumens V (ml), der Zeitspanne $T_a$, die zwischen der Erzeugung des Voralarms und dem Leerlaufen der Infusionsspritzenpumpe liegt, und des Multiplikators (Zyklusfaktors) P zur Umrechnung von Minuten auf die Takteinheit des Mikroprozessors.

Nach dem Einlesen der genannten Daten in den Mikroprozessor wird geprüft, ob der Wegsensor aktiviert ist. Wenn dies nicht der Fall ist, wird die Prüfung anschliessend immer wieder von neuem durchgeführt. Ist der Wegsensor aktiviert, so wird die Restinfusionszeit T nach der Formel

$$T = (X/R) \cdot 60 \cdot P$$

in den Zeiteinheiten des Arbeitstaktes berechnet. Ausserdem erfolgt die Umsetzung der Zeitspanne $T_a$ der Voreilung des Voralarms gegenüber der Endabschaltung in Takteinheiten des Mikroprozessors durch Multiplizieren mit dem Zyklusfaktor P $(S^{-1})$.

In der nachfolgenden Stufe wird die Restinfusionszeit mit jedem Takt verringert und anschliessend wird geprüft, ob die Restinfusionszeit T kleiner oder gleich der Zeit $T_A$ des Voralarms geworden ist. Wenn die Restinfusionszeit T grösser ist als die Zeitspanne der Voreilung des Voralarms, so wird die Zeitzählung unverändert fortgesetzt. Ist die Restinfusionszeit kleiner geworden als die Zeitspanne der Voreilung des Voralarms so wird der Alarmgeber 25 in Funktion gesetzt. Die Zeitzählung wird nun solange fortgesetzt bis die Restinfusionszeit T Null geworden ist. Wenn dies der Fall ist, erfolgt die Abschaltung der Antriebsvorrichtung und die Beendigung des Rechenvorgangs. Die Infusionsspritze ist nun vollständig leer.

Ein besonderer Vorteil des beschriebenen Verfahrens besteht darin, dass mit dem Mikroprozessor nicht nur die Auslösung des Voralarms gesteuert wird, sondern auch die Endabschaltung. Daher ist ein gesonderter Endabschalter, wie er bei den bekannten Infusionsspritzenpumpen Verwendung findet, nicht erforderlich.

## Patentanspruch

1. Verfahren zur Auslösung von Voralarm bei einer Infusionsspritzenpumpe, bei welchem ein Schlitten (13), der zum Ausdrücken einer Infusionsspritze (14) mit vorgegebener Infusionsrate (R) verschoben wird, vor Erreichen seiner Endstellung einen Sensor (21) aktiviert, dessen Stellung ein Restvolumen (X) der Infusionsspritze (14) definiert, wobei in Abhängigkeit von der Sensoraktivierung Voralarm ausgelöst wird, dadurch gekennzeichnet, dass das Restvolumen (X), die Infusionsrate (R) und eine Zeitspanne (Ta), die zwischen der Erzeugung des Voralarms und dem Infusionsende liegt, in einen Mikroprozessor (23) eingegeben werden und dass der Mikroprozessor aus dem Restvolumen (X) und der Infusionsrate (R) die Restinfusionszeit bei Sensoraktivierung berechnet und nach Ablauf einer Zeit, die mit der Aktivierung des Sensors (21) beginnt und der Differenz zwischen der Restinfusionszeit bei Sensoraktivierung und der vorgewählten Zeitspanne (Ta) entspricht, den Voralarm auslöst.

## Claim

Method for triggering an early alarm signal in an infusion syringe pump wherein a slide (13) being displaced by a predetermined infusion rate (R) for evacuating an infusion syringe (14) activates, prior to reaching its final position, a sensor (21) whose position defines a residual volume (X) of the infusion syringe (14), the early alarm signal being triggered responsive to the sensor activation, characterized in that the residual volume (X), the infusion rate (R) and the time period (Ta) between the generation of the early alarm and the end of the infusion are entered into a microprocessor (23) and that from the residual volume (X) and the infusion rate (R), the microprocessor calculates the remaining infusion time upon the sensor activation to trigger the early alarm signal upon the lapse of a time period beginning with the activation of the sensor (21) and corresponding to the difference between the remaining infusion time upon the sensor activation and the preselected time interval (Ta).

## Revendications

1. Procédé de déclenchement d'une alarme précoce dans une pompe-seringue d'injection, selon lequel un curseur (13), que l'on déplace pour refouler le produit à injecter à un débit d'injection prédéterminé (R) hors d'une seringue d'injection (14), active avant d'avoir atteint sa position d'extrémité, un capteur (21), dont la position définit un volume résiduel (X) dans la seringue d'injection (14), une alarme précoce étant déclenchée en fonction de l'activation du capteur, caractérisé en ce que le volume résiduel (X), le débit d'injection (R) et un intervalle de temps (Ta), qui se déroule entre la production de l'alarme précoce et la fin de l'injection, sont introduits dans un microprocesseur (23) et en ce que le microprocesseur calcule, à partir du volume résiduel (X) et du débit d'injection (R), la durée restante d'injection lors de l'activation du capteur et déclenche l'alarme précoce après l'écoulement d'un intervalle de temps qui commence lors de l'activation du capteur (21) et correspond à la différence entre la durée résiduelle d'injection lors de l'activation du capteur et l'intervalle de temps (Ta) présélectionné.

FIG.1

FIG.2